# EUROPEAN PATENT APPLICATION

(11) **EP 4 442 325 A1**
(43) Date of publication of application: **09.10.2024**
(21) Application number: 22901120.0
(22) Date of filing: 18.11.2022
(51) Int. Cl.: A61Q 1/00, A61Q 19/00, A61K 8/49, A61K 8/73

(54) **COSMETIC**

(30) Priority: 02.12.2021 JP 2021196472
(71) Applicant: Shiseido Company, Ltd., Chuo-ku Tokyo 104-0061 (JP)
(72) Inventor: KOMAI, Ryota, Tokyo 104-0061 (JP); OKUYAMA, Yusuke, Tokyo 104-0061 (JP)
(74) Representative: Ter Meer Steinmeister & Partner
(86) International application number: PCT/JP2022/042820
(87) International publication number: WO 2023/100687

(57) **Abstract**

Provided is a cosmetic excellent in freshness during use and usability during or after application.

A cosmetic according to the present invention includes a hydrophobically modified alkyl cellulose and an organic acid or a salt thereof represented by the following general formula (I): 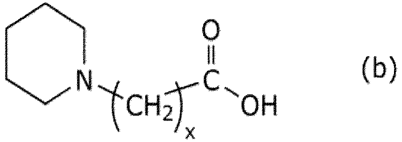 wherein n represents an integer of 2 to 5. The cosmetic may further include succinoglycan as necessary.

## Description

### TECHNICAL FIELD

The present invention relates to a cosmetic capable of achieving both freshness and usability during or after application.

### BACKGROUND ART

Cosmetics applied directly to skin are often required to be fresh. In particular, in cosmetics including a relatively large amount of oil component, various improving ways have been studied in order to achieve a fresh usability. For example, it has been studied to use a hydrophobically modified alkyl cellulose in an oil-in-water type cosmetic in order to improve freshness when an oil component is increased (Patent Literature 1).

However, according to the study of the present inventors, when a hydrophobically modified alkyl cellulose is used in various cosmetics, freshness can be greatly improved, but sliminess during application, stickiness after application, and the like may occur, and improvement thereof has been desired.

### CITATION LIST

### PATENT LITERATURE

Patent Literature 1: JP 6279457 B2

### SUMMARY OF THE INVENTION

In order to solve the above problems, it has been desired to develop a technique capable of maintaining other usability at a high level while improving freshness by blending hydrophobically modified alkyl cellulose in a cosmetic.

According to the present invention, the following invention is provided.
[1] A cosmetic including:
   (A) a hydrophobically modified alkyl cellulose; and
   (B) an organic acid or a salt thereof represented by the following general formula (b): 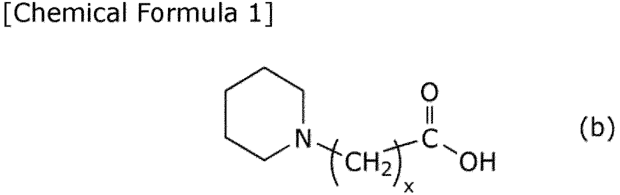
   wherein, x represents an integer of 2 to 5.
[2] The cosmetic according to [1], wherein a blending amount of the hydrophobically modified alkyl cellulose is 0.03 to 1 mass% based on a total mass of the cosmetic.
[3] The cosmetic according to [1] or [2], wherein the hydrophobically modified alkyl cellulose is hydrophobically modified hydroxypropylmethylcellulose.
[4] The cosmetic according to any one of [1] to [3], wherein a blending amount of the organic acid or the salt thereof is 0.005 to 7 mass% based on the total mass of the cosmetic.
[5] The cosmetic according to any one of [1] to [4], wherein a mass ratio of the blending amount of the hydrophobically modified alkyl cellulose to the blending amount of the organic acid or the salt thereof is 0.02 to 5.
[6] The cosmetic according to any one of [1] to [5], wherein the organic acid is 1-piperidinepropionic acid.
[7] The cosmetic according to any one of [1] to [6], further including succinoglycan.
[8] The cosmetic according to [7], wherein a blending amount of succinoglycan is 0.01 to 0.5 mass% based on the total mass of the cosmetic.
[9] The cosmetic according to [7] or [8], wherein a mass ratio of the blending amount of succinoglycan to the blending amount of organic acid is 1/200 to 1.

According to the present invention, there is provided a cosmetic in which use of a hydrophobically modified alkyl cellulose improves the sliminess during application, and the stickiness and the uncomfortable coating feeling after application, which may be brought about by a polymer such as cellulose or the like, while achieving freshness during use.

### BEST MODE FOR CARRYING OUT THE INVENTION

Hereinafter, embodiments of the present invention will be described in detail.

The cosmetic according to the present invention includes (A) a hydrophobically modified alkyl cellulose and (B) a specific organic acid as essential components.

### [Hydrophobically modified alkyl cellulose]

A hydrophobically modified alkyl cellulose (hereinafter, the component may be referred to as a component (A)) that can be used in the cosmetic according to the present invention is preferably an alkyl cellulose hydrophobically modified with an alkyl group having 14 to 22 carbon atoms. The hydrophobically modified alkyl cellulose is a compound in which a long chain alkyl group as a hydrophobic group is introduced into a water-soluble cellulose ether derivative, and is represented by the following general formula (a): 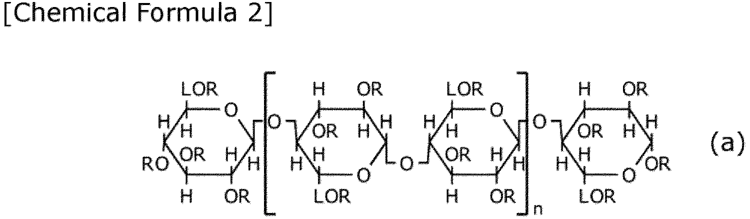 wherein R may be the same or different, and is one or more groups selected from a hydrogen atom, an alkyl group having 1 to 4 carbon atoms, a group -[CH₂CH(CH₃)O]ₘ-H (wherein m is an integer of 1 to 5, preferably 1 to 3), a group -CH₂CH₂OH, and a group -CH₂CH(OH)CH₂OR' (wherein R' is an alkyl group having 14 to 22 carbon atoms), but necessarily includes the group -CH₂CH(OH)CH₂OR'; and L is a group -(CH₂)_{q}- (q is an integer of 1 to 3, and preferably 1) and n is an integer of generally 100 to 10000, and preferably 500 to 5000.

A method for producing a hydrophobically modified alkyl cellulose of the formula (a) can be generally obtained by bringing a compound for introducing a long chain alkyl group having 14 to 22 carbon atoms, specifically, a long chain alkylglycidyl ether of the following formula (a-i) into contact with a base water-soluble cellulose ether derivative, specifically, methylcellulose (R is a hydrogen atom or a methyl group), ethylcellulose (R is a hydrogen atom or an ethyl group), propylcellulose (R is a hydrogen atom or a propyl group), butylcellulose (R is a hydrogen atom or a butyl group), hydroxypropylcellulose [R represents a hydrogen atom or a hydroxypropyl group (group - [CH₂CH(CH₃)O]ₘ-H (wherein m is an integer of 1 to 5, and preferably 1 to 3))], hydroxypropylmethylcellulose (R represents a hydrogen atom, a methyl group, or a hydroxypropyl group (the same as above)), or the like in the presence of an alkali catalyst: 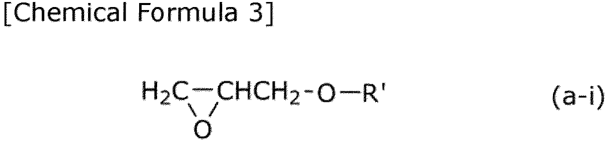 wherein R' is an alkyl group having 14 to 22 carbon atoms.

The content of the group -CH₂CH(OH)CH₂OR' introduced into the hydrophobically modified alkyl cellulose of the present invention is preferably about 0.1 to 5.0 mass% with respect to the entire hydrophobically modified alkyl cellulose. In order to achieve such a content, the water-soluble cellulose ester derivative and a long-chain alkylglycidyl ether may be produced by appropriately selecting molar ratio, reaction time, types of alkali catalyst, and the like during the reaction. After the above reaction, purification steps such as neutralization, filtration, washing, drying, and sieving of the reaction product may be performed.

Among the above-mentioned water-soluble cellulose ether derivatives, it is particularly preferable to select hydroxypropylmethylcellulose (thereby, R in the formula (a) becomes any one of four kinds of groups of a hydrogen atom, a methyl group, a group -[CH₂CH(CH₃)O]ₘH, and a group-CH₂CH(OH)CH₂OR', q of the group L becomes 1, and the group L becomes a methylene group).

Furthermore, R' in the long-chain alkylglycidyl ether of the formula (a-i) is an alkyl group having 14 to 22 carbon atoms, preferably an alkyl group having 14 to 20 carbon atoms, and more preferably a stearyl group having 18 carbon atoms (-C₁₈H₃₇). When the number of carbon atoms of the alkyl group R' is less than 14 or 23 or more, an emulsion stability by the resulting hydrophobically modified alkyl cellulose may be insufficient, and thus caution is required.

A weight average molecular weight of the hydrophobically modified alkyl cellulose is preferably 100,000 to 1000,000, more preferably 300,000 to 800,000, and still more preferably 550,000 to 750,000.

In the present invention, stearoxyhydroxypropylcellulose is most preferably used as the hydrophobically modified alkyl cellulose, and a commercially available product can also be used. For example, SANGELOSE 90L (label name: hydrophobized hydroxypropylmethylcellulose; manufactured by Daido Chemical Industry Co., Ltd.), Natrosol Plus 330cs (manufactured by Ashland), Polysurf 67cs (manufactured by Ashland), and the like.

A blending ratio of the hydrophobically modified alkyl cellulose (A) in the emulsion cosmetic of the present invention is 0.03 to 1 mass%, preferably 0.1 to 0.8 mass%, and more preferably 0.2 to 0.6 mass%. By setting the blending ratio of the hydrophobically modified alkyl cellulose within such a range, it is possible to feel more freshness during application of the cosmetic, and it is possible to suppress feeling of uncomfortable stickiness after application and feeling of sliminess during application.

### [Organic acid or salt thereof]

The cosmetic according to the present invention includes a specific organic acid or a salt thereof. This organic acid is represented by the following general formula (b): 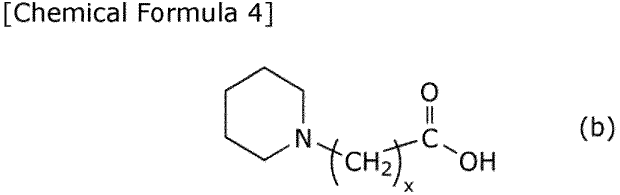 wherein x represents an integer of 2 to 5.

The organic acid is, depending on the number of x, 1-piperidinepropionic acid (x = 2), 4-(1-piperidinyl)butanoic acid (x = 3), 5-(1-piperidinyl)pentanoic acid (x = 4), or 6-(1-piperidinyl)hexanoic acid (x = 5), and among these, 1-piperidinepropionic acid has a remarkable effect of improving the sliminess during coating, the stickiness after coating, and the coating feeling caused by hydrophobically modified alkyl cellulose.

The salt of such an organic acid is not particularly limited, and examples of the inorganic salt include hydrochloride, sulfate, phosphate, hydrobromide, sodium salt, potassium salt, magnesium salt, calcium salt, and ammonium salt. Examples of the organic salt include acetate, lactate, maleate, fumarate, tartrate, citrate, methanesulfonate, p-toluenesulfonate, triethanolamine, diethanolamine, and amino acid salts.

The blending amount of the organic acid or the salt thereof is not particularly limited, but is preferably 0.005 to 7 mass%, and more preferably 1 to 3 mass%, based on the total mass of the cosmetic. When the blending amount of the organic acid or the salt thereof is within this range, it is possible to provide a cosmetic that can suppress a feeling of irritation, has an excellent moisturizing feeling, has less sticky feeling, and has an excellent usability.

In addition, in the present invention, in order to maintain an optimum balance between freshness by the hydrophobically modified alkyl cellulose and an effect of improving the usability by the organic acid or a salt thereof, a mass ratio (A/B) of a blending amount (A) of the hydrophobically modified alkyl cellulose to the blending amount (B) of the organic acid or the salt thereof is preferably 0.02 to 5, and more preferably 0.15 to 1.5.

### [Other components]

The cosmetic according to the present invention includes the hydrophobically modified alkyl cellulose described above and the organic acid or the salt thereof as essential components, but may include other components as necessary.

Among such components, succinoglycan can be mentioned as a component that is preferably blended in the cosmetic product according to the present invention. The succinoglycan is generally used as a thickener and tends to cause stickiness, however, in the cosmetic according to the present invention, an appropriate amount of succinoglycan is blended to exhibit an effect of suppressing stickiness.

The succinoglycan is a kind of polysaccharide derived from microorganisms, and includes units derived from succinic acid and pyruvic acid, and acetic acid as an optional component, or salts of these acids, in addition to sugar units derived from galactose and glucose.

More specifically, succinoglycan is a water-soluble polymer including glucose unit : galactose unit : succinic acid unit : pyruvic acid unit at about 7 : 1 : 1 : 1.

The blending amount of succinoglycan is not particularly limited, but is preferably 0.01 to 0.5 mass%, and more preferably 0.02 to 0.2 mass%, based on the total mass of the cosmetic. In the cosmetic according to the present invention, when the blending amount of succinoglycan is in this range, the freshness during use is further improved, and the stickiness after application tends to be improved.

In the present invention, a mass ratio (C/B) of a blending amount (C) of succinoglycan to a blending amount (B) of organic acid or a salt thereof is preferably 0.005 to 1, and more preferably 0.0067 to 0.5 in order to maintain an optimum balance between freshness and other usability.

In addition, the cosmetic product according to the present invention is appropriately blended as necessary with components usually used in cosmetic products, pharmaceuticals, and the like, for example, water, oil components, surfactants, powders, coloring materials, alcohols, thickeners, chelating agents, silicones, antioxidants, ultraviolet absorbers, moisturizers, fragrances, various medicinal ingredients, preservatives, pH adjusting agents, neutralizing agents, and the like.

In the cosmetic according to the present invention, it is preferable to blend a large amount of water in order to form a cosmetic liquid having a whitening effect or an emulsion in particular. As the water, water used for cosmetic products, quasi-drugs, and the like can be used, and for example, purified water, ion-exchanged water, tap water, and the like can be used. The blending amount of water varies depending on the blending amount of other components, but is generally preferably 20 to 80 mass% and more preferably 30 to 70 mass% based on the total mass of the cosmetic.

The cosmetic according to the invention can include a polyhydric alcohol. Such a polyhydric alcohol can be arbitrarily selected as long as the effect of the present invention is not impaired. Specifically, the polyhydric alcohol is preferably one kind or two or more kinds selected from a group consisting of glycerin, diglycerin, polyglycerin, 1,3-butyleneglycol, ethyleneglycol, propyleneglycol, dipropyleneglycol, polyethyleneglycol, polypropyleneglycol, polyethyleneglycol/polypropyleneglycol, trimethylolethane, trimethylolpropane, erythritol, pentaerythritol, sorbitan, glucose, sorbitol, maltitol, sucrose, raffinose, hexyleneglycol, 1,2-pentanediol, and trehalose.

The polyhydric alcohol exerts a moisturizing function and a thermal sensation imparting function, and the blending amount thereof is adjusted depending on purposes. In general, the blending amount of polyhydric alcohol is preferably 1 to 30 mass%, and more preferably 5 to 20 mass%, based on the total mass of the cosmetic.

In the present invention, the cosmetic according to the present invention can include a higher alcohol, and the higher alcohol is not particularly limited as long as it can be used in fields of cosmetic products, pharmaceuticals, quasi-drugs, and the like. Specific examples thereof include lauryl alcohol, myristyl alcohol, cetyl alcohol, stearyl alcohol, and behenyl alcohol. In addition, mixtures thereof, for example, cetearyl alcohol (cetostearyl alcohol) which is a mixture of stearyl alcohol and cetyl alcohol, and the like are generally known, and can also be used.

In a case of blending the higher alcohol, one kind or two or more kinds described above can be used. In the present invention, it is preferable to use a mixture of two or more kinds of aliphatic alcohols, and it is more preferable to use such a combination that a melting point of the mixture is 60°C or higher. If the melting point is lower than 60°C, temperature stability of the system may be deteriorated depending on formulations. In the present invention, for example, a combination of stearyl alcohol and behenyl alcohol is preferable.

The blending amount of higher alcohol is preferably 0.1 to 10 mass%, and more preferably 0.5 to 5 mass% with respect to the total mass of the cosmetic.

The cosmetic according to the present invention can include a surfactant. As the surfactant, various surfactants such as nonionic surfactants, cationic surfactants, anionic surfactants, amphoteric surfactants, and silicone surfactants are known, and any surfactant selected from them can be used. The blending amount of surfactant is preferably 0.1 to 20 mass%, and more preferably 0.3 to 5 mass% with respect to the total amount of the oil-in-water type emulsified cosmetic.

The cosmetic according to the present invention can include any oil component depending on purposes. The oil component that can be used in the present invention is not particularly limited, and can be selected from those used in cosmetic products as long as the stability is not impaired. Examples of preferable oil components include polar oils such as hydrocarbon oils and ester oils, silicone oils, and liquid oil-and-fat.

Specifically,
examples of the hydrocarbon oil include liquid paraffin, squalane, squalene, paraffin, isoparaffin, ceresin, isohexadecane, and isododecane;
examples of polar oils such as ester oils include pentaerythrityltetraethylhexanoate, cetylethylhexanoate, jojoba oil, di(phytosteryl/octyldodecyl)lauroylglutamate, triisostearate, glyceryldiisostearate, triethylhexanoin, phytosteryl/behenyl dimer dilinoleate, phytosteryl/isostearyl/cetyl/stearyl/isostearyl/cetyl/stearyl/behenyl dimer dilinoleate, isopropylpalmitate, macadamia nut fatty acid phytosteryl, pentaerythrityl tetrabehenate/benzoate/ethylhexanoate, ethylhexylpalmitate, myristylmyristate, isopropylmyristate, tripropyleneglycoldipivalate, diisopropylsebacate, and isodecylneopentanoate;
examples of the silicone oil include chain silicones such as dimethylpolysiloxane, methylphenylpolysiloxane, and methylhydrogenpolysiloxane; cyclic silicones such as octamethylcyclotetrasiloxane, decamethylcyclopentasiloxane, and dodecamethylcyclohexasiloxane; silicone resins forming a three-dimensional network structure; and silicone rubbers; and
examples of the liquid oil-and-fat include linseed oil, camellia oil, macadamia nut oil, corn oil, mink oil, olive oil, avocado oil, sasanqua oil, castor oil, safflower oil, rapeseed oil, soybean oil, peanut oil, triglycerin, glycerintrioctanoate, glycerintrioctanoate, and glycerintriisopalmitate. In a case where these oil components are included, the oil-in-water type cosmetic is preferable.

The blending amount of oil component is not particularly limited, but is preferably 5 to 40 mass% with respect to the total amount of the oil-in-water type emulsified cosmetic.

The cosmetic according to the invention can be formulated with further components depending on purposes. Examples of such components include amino acids, bactericides, anti-inflammatory agents, astringents, animal and plant extracts, vitamins other than vitamin C and derivatives thereof, chelating agents, dyes, antioxidants, preservatives, and perfumes.

### [Cosmetic]

The cosmetic according to the present invention includes the essential components described above, but can be used in all dosage forms that can be taken by general cosmetics. For example, specifically, it can be a dosage form such as a serum, an emulsion, a lotion, a cream, or an aerosol. In any of the dosage forms, it is possible to provide a cosmetic having both less feeling of irritation and excellent usability while achieving a whitening effect.

### Examples

### [Examples 1 to 11, and Comparative Examples 1 and 2]

Cosmetics of the examples and the comparative examples were prepared with the formulations shown in Table 1.

Furthermore, these cosmetics were evaluated for the freshness during use, the stickiness after application, the sliminess during application, and the coating feeling after application (presence or absence of uncomfortable coating feeling) according to the following criteria.

### [Evaluation method]

An actual use test by 20 specialized panelists was performed using the cosmetic of each example. Each expert panelist evaluated each test item based on the following evaluation point criteria, and ranked the cosmetic by the sum of the evaluation points.

### Evaluation point criteria:

5 points: Excellent.
4 points: Good.
3 points: Ordinary.
2 points: Poor.
1 point: Very poor.

### Evaluation rank

A+: 90 or more points in total
A: 80 or more points in total
B: 60 points or more and less than 80 points in total
C: 40 points or more and less than 60 points in total
D: less than 40 points in total

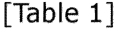

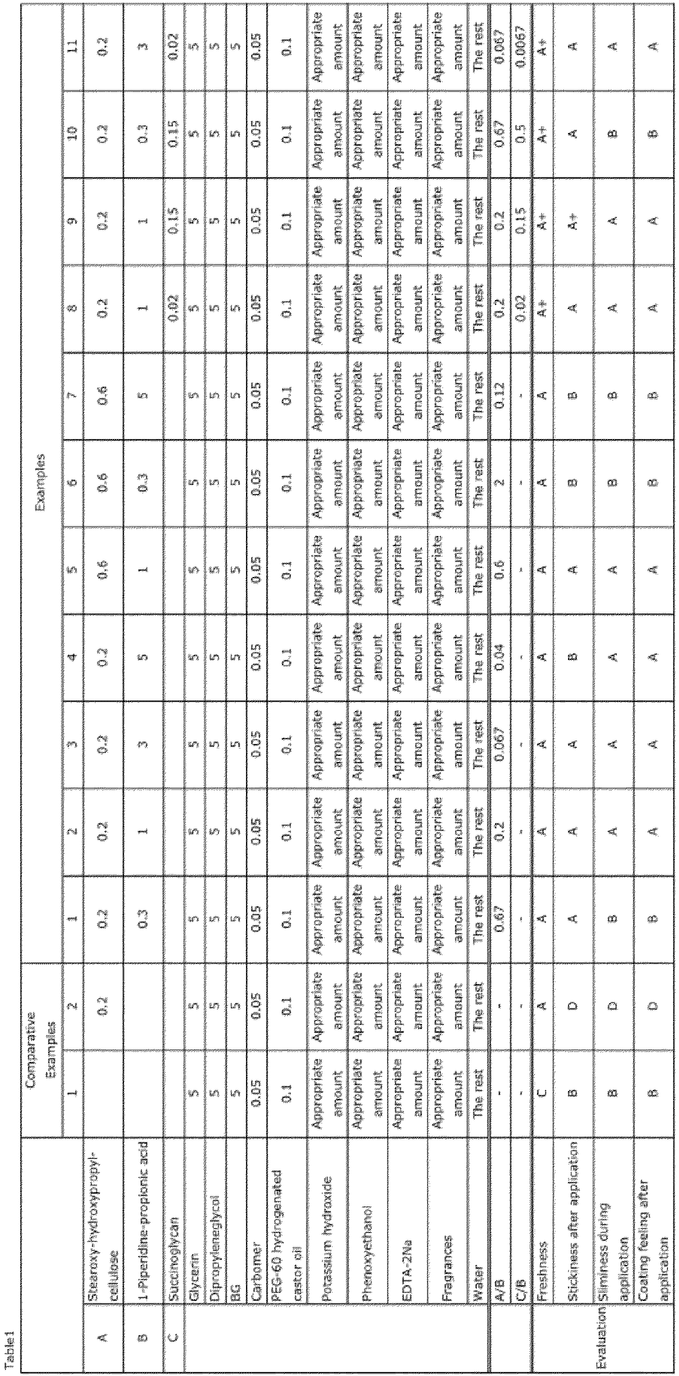

From the results of Comparative Examples 1 and 2, it is found that, although the freshness is improved by blending the hydrophobically modified alkyl cellulose, the stickiness after application, the sliminess during application, and the uncomfortable coating feeling tend to occur after application. It was found that when 1-piperidinepropionic acid, which is a specific organic acid, is combined with the hydrophobically modified alkyl cellulose, they tend to be improved, and further when succinoglucan is also combined with the hydrophobically modified alkyl cellulose, they tend to be further improved. As described above, it can be seen that the cosmetic according to the present invention has improved in term of the stickiness after application, the sliminess during application, and the uncomfortable coating feeling after application while obtaining the freshness during application.

## Claims

1. A cosmetic comprising:
(A) a hydrophobically modified alkyl cellulose; and
(B) an organic acid or a salt thereof represented by a following general formula (b): 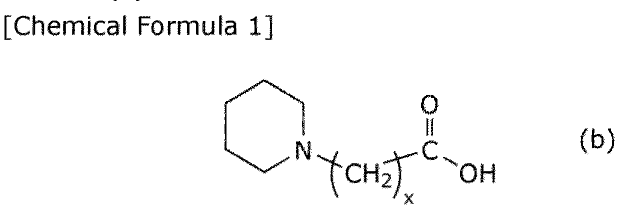
wherein x represents an integer of 2 to 5.

2. The cosmetic according to claim 1, wherein a blending amount of the hydrophobically modified alkyl cellulose is 0.03 to 1 mass% based on a total mass of the cosmetic.

3. The cosmetic according to claim 1 or 2, wherein the hydrophobically modified alkyl cellulose is hydrophobically modified hydroxypropylmethylcellulose.

4. The cosmetic according to claim 1 or 2, wherein a blending amount of the organic acid or the salt thereof is 0.005 to 7 mass% based on the total mass of the cosmetic.

5. The cosmetic according to claim 1 or 2, wherein a mass ratio of the blending amount of the hydrophobically modified alkyl cellulose to a blending amount of the organic acid or the salt thereof is 0.02 to 5.

6. The cosmetic according to claim 1 or 2, wherein the organic acid is 1-piperidinepropionic acid.

7. The cosmetic according to claim 1 or 2, further comprising succinoglycan.

8. The cosmetic according to claim 7, wherein a blending amount of succinoglycan is 0.01 to 0.5 mass% based on the total mass of the cosmetic.

9. The cosmetic according to claim 7, wherein a mass ratio of the blending amount of succinoglycan to the blending amount of organic acid is 0.005 to 1.
